# EUROPÄISCHE PATENTSCHRIFT

(11) **EP 1 598 668 B1**
(45) Veröffentlichungstag und Bekanntmachung des Hinweises auf die Patenterteilung: **29.08.2007**
(21) Anmeldenummer: 05090133.9
(22) Anmeldetag: 11.05.2005
(51) Int. Cl.: G01N 33/20, G01N 21/892

(54) **Verfahren zur Ermittlung makroskopischer Einschlüsse in Metallen**
Method for detecting macroscopic inclusions in metals
Méthode pour la détection d'inclusions macroscopiques dans des métaux

(30) Priorität: 14.05.2004 DE 102004025098; 09.05.2005 DE 102005022185
(43) Veröffentlichungstag der Anmeldung: 23.11.2005
(73) Patentinhaber: Salzgitter Mannesmann GmbH, 38239 Salzgitter (DE)
(72) Erfinder: Weinberg, Matthias, Dr., 47809 Krefeld (DE)
(74) Vertreter: Meissner, Peter E.

(56) Entgegenhaltungen:
- EP-A- 0 302 003
- EP-A- 0 880 023
- "Stahllexikon" [Online] 30. Januar 2003 (2003-01-30), PETER DRÖSSER GMBH , XP002342421 Gefunden im Internet: URL:http://web.archive.org/web/20030130143 815/www.droesser.de/droesser/lexikon/s/stu fendrehprobe.html> [gefunden am 2005-08-26] Web-Archiv-Kopie der Internetseite "www.droesser.de/droesser/lexikon/s/stufen drehprobe.html" vom 30.01.2003 (s. URL) * Seite S, Absatz STUFENDREHPROBE *

## Beschreibung

Die Erfindung betrifft ein Verfahren zur Ermittlung makroskopischer Einschlüsse in Metallen gemäß dem Oberbegriff des Patentanspruches 1.

Im Bereich der Eisenlegierungen findet die Erfindung vorzugsweise Anwendung bei Stählen, die im Stranggussverfahren vergossen wurden.

Unter Metallen werden Eisen- und Nichteisenmetalle sowie deren Legierungen verstanden, die infolge ihres metallurgischen Herstellungsprozesses nicht gewünschte Verunreinigungen enthalten. Diese Verunreinigungen bestehen im Wesentlichen aus nichtmetallischen mikro-oder makroskopischen Einschlüssen. Die Höhe des Gehaltes an nichtmetallischen Einschlüssen in einem Stahl ist ein Maß für seinen Reinheitsgrad. Entsprechend der Einteilung der Einschlüsse in mikroskopische und makroskopische Einschlüsse unterscheidet man auch zwischen dem mikroskopischen und makroskopischen Reinheitsgrad.

Der mikroskopische Reinheitsgrad ist auf dem heute üblichen Niveau für viele Verarbeitungs- und Gebrauchseigenschaften ohne große Bedeutung und die Verfahren zur Beschreibung und Bewertung des mikroskopischen Reinheitsgrades sind bewährt (Stahl und Eisen 114, 1994, Nr. 7). Die mikroskopischen Einschlüsse liegen im Stahl im Regelfall gleichmäßig und fein verteilt vor. Die gängigsten und seit Jahrzehnten angewandten Verfahren zur Beschreibung des Mikro-Reinheitsgrades sind die visuelle Bewertung der Einschlüsse an einer Probe im Mikroskop und die Zuordnung zu Vergleichsbildern.

Wenngleich die Grenzen zwischen mikro- und makroskopischem Reinheitsgrad auch nicht exakt definiert sind, werden als makroskopische Einschlüsse im Folgenden Einschlüsse ≥ 20 µm bezeichnet.

Mit steigenden Qualitätsanforderungen an das Produkt wird die Bestimmung des makroskopischen Reinheitsgrades problematischer. Standardisierte Prüfmethoden büßen zunehmend an Aussagekraft ein. Die heute üblichen metallurgischen Verfahrensweisen haben dazu geführt, dass der makroskopische Reinheitsgrad auf einem sehr hohen Niveau liegt. Für die Prüfung bedeutet dies, dass zur Beschreibung des Makro-Reinheitsgrades Einschlüsse gefunden werden müssen, die als "singuläre Ereignisse" nur in sehr geringer Häufigkeit und unregelmäßig verteilt im Stahl vorliegen.

Mehr und mehr wird die Ausfallrate am Produkt - bei der Fertigung oder im Gebrauch - als Kennwert für den Reinheitsgrad herangezogen. Für die Stahlherstellung und metallurgische Entwicklungen ist diese Situation unbefriedigend, weil keine Rückkopplung zum Verbraucher besteht oder die Information viel zu spät kommt.

Bekannte Prüfmethoden zur makroskopischen Reinheitsgradbestimmung sind z. B.:
- Schwefelabzug (Baumannabdruck)
- Metallographie nach Stahleisenprüfblatt 1570
- Stufendrehprobe nach Stahleisenprüfblatt 1580
- Slime Extraction Methode (Rückstandsisolierung)
- Quantitative Metallographie an 200 cm² großen Flächen

Eine Kritik an diesen Prüfmethoden würde hier den Rahmen sprengen. Aus Sicht der Reinheitsgradentwicklung werden die bekannten Methoden der Zielsetzung einer rationellen, schnellen, kostengünstigen, zuverlässigen und quantifizierbaren Bestimmung des Reinheitsgrades nicht gerecht. Dies gilt insbesondere für die Bestimmung des makroskopischen oxidischen Reinheitsgrades, da die Einschlüsse, wie oben beschrieben, nur in sehr geringer Häufigkeit und statistisch verteilt vorliegen, wobei sich mit den bekannten Verfahren nur bedingt statistisch gesicherte Rückschlüsse auf die Erzeugungsbedingungen ermitteln lassen.

In der DE 37 36 389 C2 wird ein Verfahren zur Herstellung von Proben für die Reinheitsgradbestimmung von Metallen mittels Ultraschall beschrieben, mit dem im Vergleich zu den bekannten Verfahren wesentliche Nachteile überwunden werden.

Kerngedanke des bekannten Verfahrens ist eine Konzentrierung der Einschlüsse bzw. Verunreinigungen in bestimmten Bereichen, so dass aus der dort angetroffenen Anhäufung eine Aussage über den Reinheitsgrad eines Erzeugnisses, und insbesondere des Stranggussmaterials selbst, möglich ist. Dieses Verfahren hat gegenüber den bekannten Verfahren den Vorteil, dass ein vergleichsweise großes Probenvolumen untersucht und eine statistisch abgesicherte Aussage über den aktuellen Reinheitsgrad erreicht wird.

Voraussetzung ist jedoch in der Regel, dass Proben in einer bestimmten Weise umgeformt werden müssen, um die Einschlüsse so zu verformen, dass sie eine günstige Reflexionsfläche für den Ultraschall bieten. Die Oberflächen der so umgeformten Proben müssen anschließend zur Durchführung der Ultraschallprüfung entsprechend vorbereitet werden.

Dieses Verfahren hat daher den Nachteil einer aufwändigen Probenherstellung und langer Probenlaufzeiten.

Ein weiterer Nachteil besteht darin, dass dieses Verfahren nur indirekt arbeitet, da der Reinheitsgrad, insbesondere der makroskopische oxidische Reinheitsgrad, indirekt aus der Schalllaufzeit abgeleitet wird, und die Schalllaufzeit ist abhängig von der Probenvorbereitung und vom Einschlusstyp.

Die Beurteilung des makroskopischen oxidischen Reinheitsgrades von Stählen ist mit diesem bekannten Verfahren sehr zeitaufwändig und teuer. Für die Kontrolle und die Weiterentwicklung der Stahlherstellungsprozesse ist eine zeitnahe und kostengünstige Beurteilung des makroskopischen Reinheitsgrades wünschenswert.

Aus der EP 0 880 023 A1 ist ein Verfahren zur automatischen Detektion von Oberflächenfehlern an Stranggussbrammen bekannt.

Hier werden die nach dem Abschleifen der Brammenoberfläche optisch detektierbaren Fehler als Bildinformation einem Bildverarbeitungsprozessor zugeleitet und von einem an die Maschinensteuerung der Schleifmaschine angeschlossenen, ein Vergleichs- und Auswertemodul besitzenden Rechner eingelesen und mit abgespeicherten Bildern typischer Oberflächenfehler verglichen. Je nach Größe der Fehler werden diese anschließend entweder direkt nachbearbeitet oder in einer Oberflächendatei abgespeichert. Eine Ermittlung von makroskopischen Einschlüssen im Stahl ist mit diesem Verfahren nicht möglich.

Aufgabe der Erfindung ist es, ein Verfahren zur Ermittlung makroskopischer Einschlüsse in Metallen, insbesondere von im Stranggussverfahren vergossenen Stählen anzugeben, das eine kostengünstigere und schnellere Beurteilung des makroskopischen Reinheitsgrades ermöglicht.

Diese Aufgabe wird nach dem Oberbegriff in Verbindung mit den kennzeichnenden Merkmalen des Anspruchs 1 gelöst. Vorteilhafte Weiterbildungen sind Gegenstand von Unteransprüchen.

Nach der Lehre der Erfindung wird zur Lösung dieser Aufgabe ein Verfahren verwendet, bei dem mittels eines spanhabhebenden Verfahrens von einem Probenrohling eine eine statistisch gesicherte Auswertung ermöglichende, abgearbeitete Probenoberfläche (Schnittfläche) erzeugt wird, wobei während des Spanabhebens die jeweils neu erzeugte Schnittfläche hinsichtlich vorhandener makroskopischer Einschlüsse kontinuierlich optisch erfasst und ausgewertet wird.

Mit Hilfe von Berechnungsmethoden, die aus der quantitativen Gefügeanalyse/Stereologie bekannt sind, können Konzentration und Größenverteilung makroskopischer Einschlüsse aus Messwerten ermittelt werden, die aus der ebenen Fläche (Probenober- bzw. Schnittfläche) gewonnen wurden.

Vorteil dieses Verfahrens ist, dass eine sehr kostengünstige, schnelle und bessere Beurteilung, des makroskopischen, insbesondere des oxidischen Reinheitsgrades, ermöglicht wird, wobei die Konzentration makroskopischer Einschlüsse im Stahl auf Basis der Untersuchung einer repräsentativen Probe aus dem stranggegossenen Stahl beschrieben wird.

Aufwändige Probenvorbereitungen durch Glühen und Walzen entfallen, so dass die Probenlaufzeit deutlich reduziert wird.

Durch die kontinuierliche optische Erfassung und Auswertung der Bildinformationen wird zudem vorteilhaft eine Klassifizierung vorhandener makroskopischer Einschlüsse möglich, mit der eine schnelle Beurteilung des makroskopischen Reinheitsgrades des Stahles erfolgen kann.

Gleichzeitig besteht durch die sehr schnelle Ermittlung des Reinheitsgrades die Möglichkeit zeitnah Korrekturmaßnahmen im Erzeugungsprozess vorzunehmen. Insbesondere bei Stählen mit hohen und höchsten Anforderungen an den makroskopischen Reinheitsgrad ("High End"- Stähle) wird die Qualitätssicherung deutlich verbessert.

Die Erfindung basiert auf der in der DE 37 36 389 C2 beschriebenen grundlegenden Untersuchung zur Lage des Einschlussbandes in Bogensträngen.

Kerngedanke der Erfindung ist, an einem aus diesem Einschlussband entnommenen Probenrohling durch spanabhebende Bearbeitung eine statistisch ausreichend große Probenoberfläche zu erzeugen, mit der die im Einschlussband unregelmäßig verteilten oxidischen Einschlüsse über ein Bildanalyseverfahren statistisch abgesichert ermittelt werden können.

Im Gegensatz zur Beurteilung des mikroskopischen Reinheitsgrades, werden hierbei wesentlich größere Probenflächen analysiert. Zur Bestimmung des makroskopischen Reinheitsgrades an einer Stranggussbramme wird für eine statistisch abgesicherte Aussage beispielsweise eine Probenoberfläche von ca. 50 m² erzeugt und analysiert.

Als spanabhebende Verfahren kommen sowohl das Drehen, Fräsen und auch Hobeln in Betracht, wobei die Geometrie des Proberohlings dem spanabhebenden Verfahren angepasst ist. Bei den heute verfügbaren Hochleistungs-Zerspanungsverfahren können auch die zur Detektion von makroskopischen Einschlüssen erforderlichen großen Zerspanungsvolumina im Hinblick auf eine zeitnahe Beurteilung des Reinheitsgrades problemlos erzeugt werden.

Über das Bildanalyseverfahren werden dann die optisch erkennbaren nichtmetallischen makroskopischen Einschlüsse ausgezählt, bewertet und eine Größenverteilung der Einschlüsse vorgenommen. Eine Detektion von nichtmetallischen Einschlüssen in der Größe von bis zu 20 µm kann mit den zur Zeit verfügbaren Bildverarbeitungssystemen erreicht werden.

Ein großer Vorteil des erfindungsgemäßen Verfahrens ist außerdem, dass es in sehr einfacher Weise automatisiert werden kann, was zu einer weiteren Kostenreduzierung und Verkürzung der Probenlaufzeit und damit zu einer zeitnahen Beurteilung des Erzeugungsprozesses führt.

Weitere Merkmale, Vorteile und Einzelheiten der Erfindung ergeben sich aus der nachfolgenden Beschreibung der einzigen **Figur.**

Dargestellt ist ein aus einem Einschlussband eines Stranggussabschnittes entnommener Probenrohling 1, hier dargestellt als Rundstab, der mittels eines Drehmeißels 2 spanabhebend in Längsachse der Probe bearbeitet wird.

Die erzeugte Schnittfläche auf der Oberfläche des Probenrohlings 1 wird über eine mit einem Linsensystem bestückte Kamera 3 kontinuierlich optisch erfasst und die Daten an ein Bildanalysesystem 4 übertragen.

Über das Bildanalysesystem 4 werden die erkennbaren nichtmetallischen Einschlüsse ausgezählt, bewertet und eine Größenverteilung der Einschlüsse vorgenommen. Das Detektionsvermögen von nichtmetallischen Einschlüssen ist dabei abhängig von der Qualität der Linsensysteme und der Bildanalyse. Interne Untersuchungen haben gezeigt, dass sich mit diesem System zur Zeit Einschlüsse bis etwa 20 µm detektieren lassen.

### Bezugszeichenliste

| **Nr.** | **Bezeichnung** |
|---|---|
| 1 | Probenrohling |
| 2 | Drehmeißel |
| 3 | Kamera |
| 4 | Bildanalysesystem |

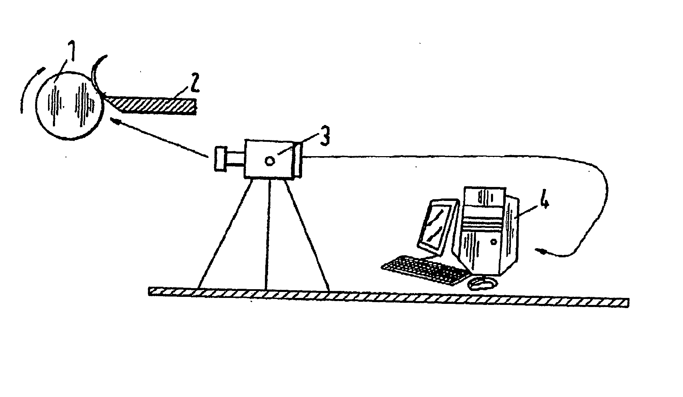

## Patentansprüche

1. Verfahren zur Ermittlung makroskopischer Einschlüsse in Metallen, insbesondere von im Stranggießverfahren vergossenen Stählen, bei denen aus einem Gusserzeugnis oder einem bereits verformten Erzeugnis ein Probenrohling ausgeschnitten wird, wobei die Längserstreckung des Probenrohlings parallel zur Gießachse bzw. der Hauptverformungsrichtung des verformten Erzeugnisses liegt,
**dadurch gekennzeichnet,**
**dass** mittels eines spanabhebenden Verfahrens vom Probenrohling eine eine statistisch gesicherte Auswertung ermöglichende, abgearbeitete Probenoberfläche, z.B. eine Schnittfläche, erzeugt wird, wobei während des Spanabhebens die jeweils neu erzeugte Schnittfläche hinsichtlich vorhandener makroskopischer Einschlüsse kontinuierlich optisch erfasst und ausgewertet wird.

2. Verfahren nach Anspruch 1
**dadurch gekennzeichnet,**
**dass** von der Schnittfläche der Probe nichtmetallische, insbesondere oxidische Einschlüsse, im Hinblick auf deren Größenverteilung analysiert und ausgewertet werden.

3. Verfahren nach Anspruch 1 und 2
**dadurch gekennzeichnet,**
**dass** das Verfahren automatisiert abläuft.

4. Verfahren nach einem der Ansprüche 1 - 3
**dadurch gekennzeichnet,**
**dass** als spanabhebendes Verfahren das Drehen angewendet wird.

5. Verfahren nach einem der Ansprüche 1-3
**dadurch gekennzeichnet,**
**dass** als spanabhebendes Verfahren das Fräsen angewendet wird.

6. Verfahren nach einem der Ansprüche 1-3
**dadurch gekennzeichnet, dass**
als spanabhebendes Verfahren das Holben angewendet wird.

## Claims

1. A process for detecting macroscopic inclusions in metals, in particular steels cast using a continuous casting process, in which a sample blank is cut out from a cast product or an already shaped product, the longitudinal extent of the sample blank lying parallel to the casting axis or the main direction of deformation of the shaped product,
**characterised in that**
a machine-reduced sample surface, e.g. a cut surface, which permits a statistically confirmed evaluation is produced from the sample blank by means of a metal-cutting process, the newly produced cut surface each time during the metal-cutting being continuously detected optically and evaluated with respect to macroscopic inclusions which are present.

2. A process according to Claim 1, **characterised in that** non-metallic, in particular oxidic, inclusions from the cut surface of the sample are analysed and evaluated with respect to their size distribution.

3. A process according to Claims 1 and 2, **characterised in that** the process takes place automatically.

4. A process according to one of Claims 1 to 3, **characterised in that** turning is used as the metal-cutting process.

5. A process according to one of Claims 1 to 3, **characterised in that** milling is used as the metal-cutting process.

6. A process according to one of Claims 1 to 3, **characterised in that** planing is used as the metal-cutting process.

## Revendications

1. Procédé pour la détection d'inclusions macroscopiques dans des métaux, en particulier dans des aciers coulés par coulée continue, dans lesquels on prélève une ébauche d'échantillon dans un produit coulé ou dans un produit déjà formé, l'étendue longitudinale de l'ébauche d'échantillon étant parallèle à l'axe de coulée ou au sens de formage principal du produit formé,
**caractérisé en ce que**, au moyen d'un procédé d'enlèvement de copeaux, on produit à partir de l'ébauche d'échantillon une surface d'échantillon, par ex. une surface de coupe, obtenue par enlèvement de matière et permettant une évaluation statistiquement sûre, la surface de coupe nouvellement produite étant observée optiquement et évaluée en continu en ce qui concerne la présence d'inclusions macroscopiques, pendant l'enlèvement de copeaux.

2. Procédé selon la revendication 1,
**caractérisé en ce qu'**à partir de la surface de coupe de l'échantillon, on analyse et évalue les inclusions non métalliques, en particulier par voie d'oxydation, en ce qui concerne leur répartition dimensionnelle.

3. Procédé selon les revendications 1 et 2,
**caractérisé en ce que** le procédé se déroule de manière automatique.

4. Procédé selon l'une des revendications 1 à 3,
**caractérisé en ce qu'**on utilise le tournage comme procédé d'enlèvement de copeaux.

5. Procédé selon l'une des revendications 1 à 3,
**caractérisé en ce qu'**on utilise le fraisage comme procédé d'enlèvement de copeaux.

6. Procédé selon l'une des revendications 1 à 3,
**caractérisé en ce qu'**on utilise le planage comme procédé d'enlèvement de copeaux.
